# EUROPEAN PATENT APPLICATION

(11) **EP 4 349 247 A1**
(43) Date of publication of application: **10.04.2024**
(21) Application number: 23202273.1
(22) Date of filing: 06.10.2023
(51) Int. Cl.: A61B 5/00, A61B 5/0205, A61B 5/024, A61B 5/08, A61B 5/11, A61B 5/113, A61B 7/00, A61B 7/02, A61B 7/04

(54) **A SYSTEM AND METHOD FOR CONTACTLESS NON-INTRUSIVE MONITORING OF PHYSIOLOGICAL CONDITIONS THROUGH ACOUSTIC SIGNALS**

(30) Priority: 08.10.2022 IN 202241057654
(71) Applicant: Turtle Shell Technologies Pvt Ltd, Bengaluru Karnataka 560038 (IN)
(72) Inventor: SARAN, Vibhor, 560038 Bengaluru (IN); SINGH, Vishwa, 560038 Bengaluru (IN); KAUSHIK, Pavan, 560038 Bengaluru (IN); PARCHANI, Gaurav, 560038 Bengaluru (IN); KADAMBI, Pooja, 560038 Bengaluru (IN)
(74) Representative: Vidon Brevets & Stratégie

(57) **Abstract**

The present invention discloses a system and method for non-intrusive monitoring and prediction of cardiac function of a user. The system (100) comprises a sensor device (102), a data capturing device (104), a data receiver module (106), an acoustic engine (108) and user devices (110), which communicate by using communication network (112). The sensor device (102) comprises a sensor array to capture micro-vibrations of physiological parameters of a user through a surface/mattress under which the sensor device (102) is positioned. The acoustic engine (108) converts the digital time-based vibration signals into frequency-based acoustic outputs such as physiological condition acoustic signals. Further, the acoustic engine (108) is configured to determine the user's current and future health issues based on the physiological condition acoustic signals. does not require trained medical practitioners to analyze the vibration signal of the user to determine the physiological conditions of the user.

## Description

### FIELD OF INVENTION

The field of invention generally relates to non-intrusive monitoring of physiological conditions through acoustic signals. More specifically, it relates to a system and method for contactless non-intrusive monitoring of physiological conditions, by converting time-based vibration signals to acoustic signals, and analyzing the same.

### BACKGROUND

Remote patient monitoring is an important healthcare area that has seen increased advances. Multiple technologies are used to capture a wide range of biological data from patients by means of multiple sensors including biosensors. Such biological data is typically collected by devices provided to the patients and is further transmitted to remote patient monitoring platforms by means of a communication network.

Such remote patient monitoring systems enable healthcare providers to monitor and assess the health conditions of patients remotely, by continuously monitoring the patient's detected vitals.

Currently, existing systems do not succeed in providing an efficient solution for detection and conversion of physiological conditions into acoustic signals which can be used by a healthcare professional for monitoring and diagnosis of a patient.

Current systems provide devices which are intrusive or require a hands-on approach to function accurately, such as advanced stethoscopes. However, these systems cannot be used for continuously or remotely monitoring a patient. Additionally, these systems also require regular human input or intervention to be able to accurately determine changes in physiological conditions. Further, these devices are limited to detecting a single parameter of a user, such as heartbeats, and cannot detect any other physiological conditions of the user.

Other existing systems have tried to address this problem. However, their scope was limited to using ECG machines, speech analysis or wave radar. Disadvantageously, many of these systems are not contactless, and cannot function by analyzing a signal alone. Additionally, these systems require specialized equipment which in turn increases the size and cost of the system, and thus are not portable or easy to use in individual settings. Another disadvantage of such systems is that they require specialized skilled technicians to operate and interpret any data captured through the same.

Thus, in light of the above discussion, it is implied that there is need for a system and method for continuously monitoring the patient's health status to derive acoustic signals for the patient's physiological conditions, without human intervention, which is non-intrusive, simple and cost effective, and does not suffer from the problems discussed above.

### OBJECT OF INVENTION

The principal object of this invention is to provide a system and method system for contactless non-intrusive monitoring of one or more physiological conditions of at least one user through acoustic signals.

A further object of the invention is to provide the system and method for converting a user's body vibrations into a created acoustic signal / representation which can be used to determine health and the one or more physiological conditions of the at least one user.

Another object of the invention is to provide the system and method for conversion of a mechanical or force-base signal (such as vibration, micro-vibration, ballistocardiograph (BCG), seismo-cardiographs, and impedance signals) to produce the created acoustic signal such as an audio file.

Another object of the invention is to provide the system and method for conversion of a vibration signal of a user to produce the created acoustic signal such as an audio file for monitoring the at least one user's physiological condition.

Another object of the invention is to provide the system and method for conversion of a vibration signal of the at least one user to the created acoustic signal which comprises combining or layering of at least one of multiple signals, layers and digital biomarkers that can depict clinical issues related to the one or more physiological conditions and bio-markers of the user's body, such as low ejection fraction, high ejection fraction, heart murmurs, lung crackles, coughs, wheezing, and fluid in any organs, among others.

Another object of the invention is to provide a simple, autonomous and cost-effective system and method for non-intrusive monitoring of one or more physiological conditions of a user by using an acoustic engine.

Another object of the invention is to provide the system and method for producing an easier representation of vibrations or micro-vibrations for automated interpretation of one or more physiological conditions of the at least one user.

Another object of the invention is to provide the system and method for non-intrusive monitoring of one or more physiological conditions through acoustic signals, which does not require trained medical practitioners to spend time and effort to analyze the vibration signal of the at least one user to determine the one or more physiological conditions of the at least one user.

Another object of the invention is to provide the system and method for non-intrusive monitoring of one or more physiological conditions through acoustic signals, which can also be used in a discrete, daily, or continuous manner, in order to autonomously detect any changes in the user's physiological conditions, without human intervention.

Another object of the invention is to provide the system and method for non-intrusive monitoring of one or more physiological conditions through acoustic signals, which is less- or non-intrusive and is very comfortable when implemented in the environment of the at least one user.

Another object of the invention is to provide the system and method for non-intrusive monitoring which eliminates any human speech and ambient sound, and enables a non-intrusive, contact-less system that works in a user's personal spaces without raising any privacy concerns.

Another object of the invention is to provide the system and method for non-intrusive monitoring by using piezoelectric elements under a dampening material like a mattress, sampling at lower rates, and software filters, which drastically reduces any high frequency sounds of human voices and other ambient noises, thus preventing any personal speech data from being recorded.

### BRIEF DESCRIPTION OF FIGURES

This invention is illustrated in the accompanying drawings, throughout which, like reference letters, indicate corresponding parts in the various figures.

The embodiments herein will be better understood from the following description with reference to the drawings, in which:
Fig. 1A depicts/illustrates a block diagram of a system for non-intrusive monitoring of physiological conditions through created acoustic signals, in accordance with an embodiment;
Fig. 1B depicts/illustrates an exemplary placement of a sensor device, in accordance with an embodiment;
Fig. 2 depicts/illustrates a detailed block diagram of components of the system for non-intrusive monitoring of physiological conditions through acoustic signals, in accordance with an embodiment;
Fig. 3 depicts/illustrates a detailed block diagram of components of an acoustic engine, in accordance with an embodiment;
Fig. 4A depicts/illustrates an exemplary vibration signal cleaned by an extraction unit, in accordance with an embodiment;
Fig. 4B depicts/illustrates a cleaned raw signal, in accordance with an embodiment;
Fig. 4C depicts/illustrates an isolated heart signal of a user, in accordance with an embodiment;
Fig. 4D depicts/illustrates an isolated breathing signal of a user, in accordance with an embodiment;
Fig. 5 illustrates a method for non-intrusive monitoring of physiological conditions through acoustic signals, in accordance with an embodiment;
Fig. 6 illustrates a method for extraction of acoustic signals and generation of created acoustic signals from a vibration signal, in accordance with an embodiment;
Fig. 7 illustrates a method for capturing data windows in a vibration signal, in accordance with an embodiment; and
Fig. 8 depicts/illustrates an exemplary placement of the sensor device and a data capturing device, in accordance with an embodiment.

### DETAILED DESCRIPTION

The embodiments herein and the various features and advantageous details thereof are explained more fully with reference to the non-limiting embodiments that are illustrated in the accompanying drawings and/or detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

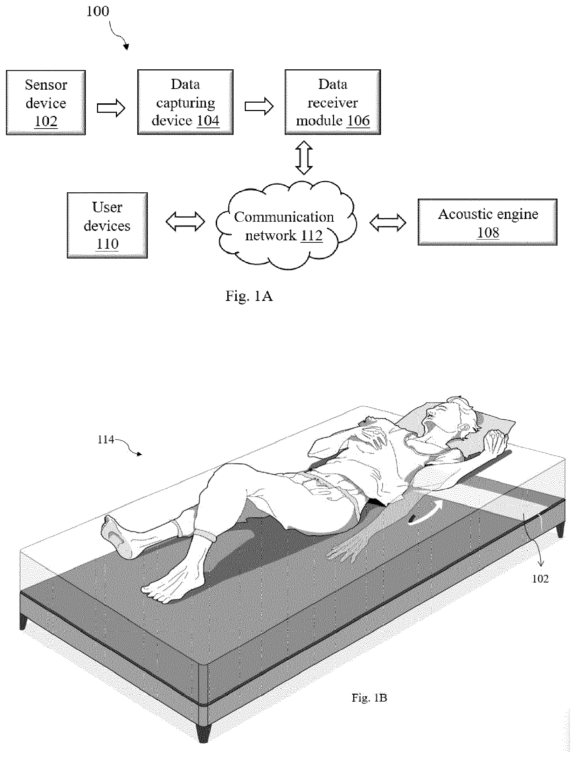

Fig. 1A depicts/illustrates a block diagram of a system 100 for non-intrusive monitoring of one or more physiological conditions through created acoustic signals, in accordance with an embodiment.

In an embodiment, the system 100 comprises at least one sensor device 102, at least one data capturing device 104, at least one data receiver module 106, an acoustic engine 108 and at least one user device 110, which may communicate by using at least one communication network 112.

In an embodiment, the at least one sensor device 102 is configured to carry out various embodiments of the present invention. The at least one sensor device 102 is placed under a surface or a comfortable medium such as a mattress to ensure maximum comfort of at least one user, and to ensure that there is no contact between the at least one sensor device 102 and the at least one user being tested/screened/monitored.

In an embodiment, the at least one sensor device 102 comprises at least one sensor array to capture micro-vibrations of one or more physiological parameters of the user's body, through the surface under which the at least one sensor device 102 is positioned.

In an embodiment, the vibrations captured by the at least one sensor device 102 comprise at least one mechanical or force-based signals such as vibrations, microvibrations, macro vibrations, ballistocardiograph (BCG vibration), seismo-cardiographs, and impedance signals associated with physiological parameters of the user's body.

In an embodiment, the vibration or microvibration signals provide a non-invasive method to measure various physiological motions of a user's body, which result from the one or more physiological conditions of the at least one user.

In an embodiment, the vibration signals comprise vibrations as well as microvibrations related to the physiological motions and conditions of a user's body, which result from the one or more physiological conditions of the at least one user.

In an embodiment, the vibration or microvibration signals may be associated with one or more physiological parameters comprising at least one of: breath, cardiac cycles, heartbeat rates, chest movements, heart movements, body movements, seizures, tremors, ticks, and respiration, among others.

Moreover, these are microvibrations are present in a subacoustic domain and are converted into the acoustic band after undergoing processing and transformation.

In an embodiment, the at least one sensor device 102 is configured to convert the one or more captured analog micro-vibrations into at least one digital data signal.

In an embodiment, the analog signals may be converted to the digital signals of any required voltage, as per the requirements of the at least one user or the hardware.

In the depicted embodiment, the at least one digital data signal may comprise micro-voltages in a range of 0.1V to 3.5V.

In an embodiment, the at least one digital data signal may comprise one or more of capacitance, impedance, micro-currents, and voltage signals.

Further, the at least one sensor device 102 is configured to communicate the at least one digital data signal to the at least one data capturing device 104, which may be positioned in the vicinity of the at least one sensor device 102.

In an embodiment, the at least one data capturing device 104 is configured to record the communicated micro-voltage acoustic data signals in a predefined chronological format for further processing. In particular, the at least one data capturing device 104 may calibrate the data signals through amplification, in order to generate an amplified signal with maximum resolution. Further, the amplified signal may also be auto-calibrated based on one or more predefined factors.

Thereafter, the amplified signal is communicated to the data receiver module 106 at regular intervals.

In an embodiment, the data receiver module 106 may comprise a stand-alone module, or may be provided within at least one of the acoustic engine 108 or the at least one user device 110.

In an embodiment, the data receiver module 106 is configured to record the communicated micro-voltage data signals in a predefined chronological format for further processing.

In an embodiment, the functionality of the acoustic engine 108 may be carried out by similar units present in the at least one user device 110.

In an embodiment, the recorded data signals are processed by various units within the acoustic engine 108 or by a user application within the at least one user device 110 configured to mirror the functions of the acoustic engine 108, in order to determine physiological condition acoustic signals of the user.

In an embodiment, as per an advantage of the system, the acoustic engine 108 may convert the digital vibration data signals (which are time-based signals) into a created frequency-based acoustic signal, such as audio files, to accurately consume and determine the current physiological conditions of the at least one user.

Subsequently, the acoustic engine 108 may analyze or compare created acoustic signals with stored physiological condition acoustic signals, in order to predict health issues of the at least one user.

In an embodiment, the stored physiological condition acoustic signals may comprise historical data or previously gathered data derived from previous users/patients. Further, the stored physiological condition acoustic signals may comprise one or more tags or descriptions of the health status or issues of the user/patient.

As an example, the stored acoustic signals may comprise signals related to at least one of physiological conditions and bio-markers of the user's body may comprise one or more conditions such as low ejection fraction, high ejection fraction, heart murmurs, lung crackles, coughs, wheezing, and fluid in any organs, among others.

Further, the created acoustic signals may be compared to the stored acoustic signals, to generate one or more alerts based on the health issues related to the one or more physiological conditions of the at least one user.

In an embodiment, one or more reports or alerts may be generated based on the currently created or future physiological condition acoustic signals.

In an embodiment, the at least one user device 110 may be used by the at least one user or a healthcare professional to continuously consume and monitor the user's physiological condition acoustic signals, and receive one or more reports or alerts related to the same.

In an embodiment, the communication network 112 may comprise wired and wireless communication, including but not limited to, GPS, GSM, WAN, LAN, Wi-fi compatibility, Bluetooth low energy as well as NFC. The wireless communication may further comprise one or more of Bluetooth (registered trademark), ZigBee (registered trademark), a short-range wireless communication such as UWB, a medium-range wireless communication such as WiFi (registered trademark) or a long-range wireless communication such as 3G/4G/5G or WiMAX (registered trademark), according to the usage environment.

Fig. 1B depicts/illustrates an exemplary placement of the at least one sensor device 102, in accordance with an embodiment.

In an embodiment, the at least one sensor device 102 is placed under a surface such as a mattress, cushion, yoga mat, bedding, futon, etc, upon which the at least one user may be seated, laying down, or sleeping. The placement of the at least one sensor device 102 may be aligned towards a sitting, leaning, resting, laying down, or sleeping position of the at least one user.

In an embodiment, the at least one sensor device 102 may be placed directly under the user.

In an embodiment, the micro-vibrations captured by the at least one sensor device 102 are converted into micro voltages. Thereafter, the micro-voltages are amplified by using a dynamic amplification ratio management module. The dynamic amplification ratio management module is configured to amplify the micro voltages based on any uncontrollable environmental factors.

In an embodiment, the captured signal may depict variations due to uncontrollable factors such as a user's weight, position and posture, as well as the material, type and thickness of the surface of medium under which the at least one sensor device 102 is positioned.

In an embodiment, such factors may spread the baseline of the signal strength over two or more orders of magnitude. Hence, the dynamic gain management module is used to provide robust data collection with high signal to noise ratio (SNR) with such challenging factors or conditions.

In an embodiment, the gain management module enables dynamic adjustment of the gain of the signal, in order to increase/decrease sensitivity of the signal. Advantageously, the dynamic management of the signal gain enables the capture of data across a wide range of uncontrollable factors.

As an example, dynamic management of the signal gain enables the capture of data from a person of weight 40-120 Kg, through a surface of mattress which is 0-50 cm thick. In an embodiment, the weight of the person and the thickness and/or material of the mattress may vary based on different use-cases.

In an embodiment, the at least one sensor device 102 may be configured to enable appropriate adjustments to the data acquisition rates to capture micro-vibrational signals from one or more other organs such as lungs and stomach; or clinically significant motions such as seizures, tremors, and ticks from the user's body, in case of rapid leg or eye movements.

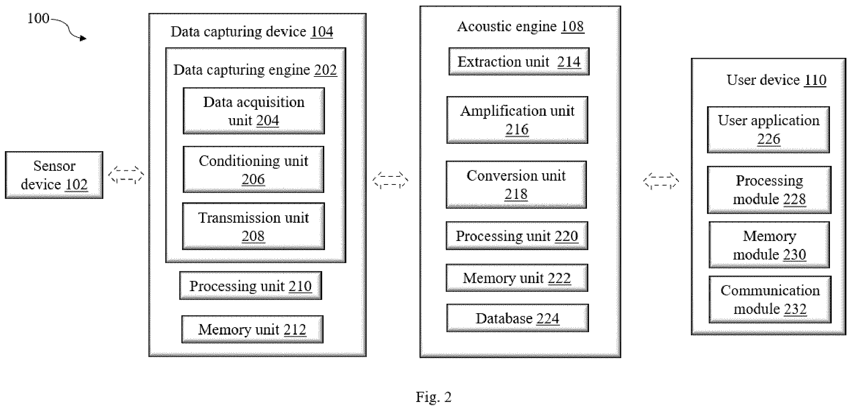

Fig. 2 depicts/illustrates a detailed block diagram of components of the system 100 for non-intrusive monitoring of physiological conditions through created acoustic signals, in accordance with an embodiment.

In an embodiment, the at least one sensor device 102 comprises at least one sensor array comprising one or more of: piezoelectric sensors, and vibroacoustic sensors, among others.

In an embodiment, the sensor array is positioned within a stabilization medium such as a holder, support or a casing. Further, the sensor array is fixed in a pattern and position which is most conducive to obtaining good quality signals from the user.

In an embodiment, the surface or comfortable medium comprises one or more of a mattress, cushion, yoga mat, bedding, futon, etc, upon which the at least one user may be seated, laying down, or sleeping.

In an embodiment, the surface or comfortable medium may comprise one or more material, comprising a spring, foam, air-based mattress/cushion and gel-based mattress/cushion.

In another embodiment, the at least one sensor device 102 may be directly positioned under the user.

In an embodiment, the at least one sensor device 102 may be 2 - 5 mm thick, and may comprise a strengthened outer cover for protecting and covering inner components of the at least one sensor device 102. The outer cover may comprise a durable material, comprising at least one of plastic, cloth, canvas, mesh, and latex, among others.

In an embodiment, the at least one sensor device 102 comprises an electromagnetic shielding fabric that encases the sensor array. This electromagnetic shielding fabric increases the signal to noise ratio of the detected signal. Advantageously, the electromagnetic shielding fabric prevents any interference or digital noise of the signal, from any erroneous noise from the environment of the at least one sensor device 102.

In an embodiment, the at least one sensor device 102 may be positioned across the body in various configurations of size and spacing.

In an embodiment, the at least one sensor device 102 may be manufactured based on various shapes and sizes, as per different user requirements. Further, different use-cases of the at least one sensor device 102 may comprise different thicknesses and shapes. In an embodiment, the shape of the at least one sensor device 102 may be polygonal, circular, triangular, square, rectangular etc.

As an example, the at least one sensor device 102 placed behind a chair cushion may vary in shape and size from the at least one sensor device 102 used underneath a mattress or a yoga mat, among others.

In an embodiment, the at least one data capturing device 104 comprises a data capturing engine 202, a processing unit 210, and a memory unit 212.

In an embodiment, the processing unit 210 of the at least one data capturing device 104 may comprise one or more of microprocessors, circuits, and other hardware configured for processing. The processing unit 210 is configured to execute instructions stored in the memory unit 212 as well as communicate with at least one of: sensor devices 102, data capturing devices 104, data receiver modules 106, and user devices 110 through an input/output module via a communication module.

In an embodiment, the memory unit 210 of the at least one data capturing device 104 comprises one or more volatile and non-volatile memory components which are capable of storing data and instructions to be executed.

In an embodiment, the data capturing engine 202 is configured to receive the data signals from the at least one sensor device 102, and process the data signals in order to derive amplified data signals.

In an embodiment, the data capturing engine 202 comprises a data acquisition unit 204, a conditioning unit 206, and a transmission unit 208.

In an embodiment, the data acquisition unit 204 is configured to receive one or more micro-voltage digital data signals captured by the at least one sensor device 102. Further, the data acquisition unit 204 is configured to record or store the received micro-voltage digital signal in a predetermined data format. The pre-defined data recording format may be based on a chronological order format. Further, the data acquisition unit 204 may store the received data signals based on one or more of: user accounts, database segments for different users, different geographical applications, and different applications of the at least one sensor device 102.

In an embodiment, the data acquisition unit 204 is configured to communicate the stored micro-voltage digital data signals to the conditioning unit 206.

In an embodiment, the data acquisition unit 204 is configured to utilize one or more encryption techniques to ensure the communication of secure, encrypted, ordered data is sent to the cloud.

In an embodiment, the conditioning unit 206 is configured to amplify the received micro-voltage digital signals in order to maximize resolution of the digital signals to obtain a desired amplified signal. The conditioning unit 206 is configured to determine the amplified signal for efficient detection of the user's physiological parameters.

In an embodiment, the conditioning unit 206 maximizes the resolution of the digital signals by ensuring prevention of any data loss which may occur due to loss of the signal.

Advantageously, the amplification and maximization of the resolution of the digital signal enables the at least one sensor device 102 to be positioned under a surface or mattress of any thickness, construction or material. Hence, the at least one sensor device 102 may be used in multiple user set-ups and environments.

In an embodiment, the conditioning unit 110 is configured to amplify the micro-voltage digital signal based on strength of the received micro-voltage digital signals.

In an embodiment, the conditioning unit 110 is configured with multiple amplification categories. In an embodiment, the conditioning unit 110 may comprise eight different amplification categories, in order to amplify the micro-voltages between a range of 15x to 2500x, which can cover various physiological conditions.

In an embodiment, the conditioning unit 110 is configured to automatically calibrate and select the amplification option based on a sensed proximity of the at least one sensor device 102 to the user, as different applications of the at least one sensor device 102 may position the at least one sensor device 102 at different distances from the body of the user.

Further, the conditioning unit 110 is configured to communicate the optimized amplified signal to the transmission unit 208.

In an embodiment, the transmission unit 208 is configured to transmit the amplified micro-voltage digital signal to one or more of the acoustic engines 108, a database 118, and the data receiver module 106.

The amplified micro-voltage digital signal may be transmitted to the through the communication network 112.

In an embodiment, the acoustic engine 108 is configured to convert the digital time-based signals into frequency-based acoustic outputs such as physiological acoustic signals.

In an embodiment, the physiological acoustic signals comprise one or physiological conditions such as low ejection fraction, high ejection fraction, heart murmurs, lung crackles, coughs, wheezing, and fluid in any organs, among others.

Further, the acoustic engine 108 is configured to determine the user's current and future health issues based on the physiological condition acoustic signals. Subsequently, the acoustic engine 108 is configured to generate one or more reports and alerts based on the determined physiological condition acoustic signals.

In an embodiment, the alerts are based on identified health issues related to the one or more physiological conditions of the user's body.

In an embodiment, the acoustic engine 108 comprises an extraction unit 214, an amplification unit 216, a conversion unit 218, a processing unit 220, a memory unit 222 and a database 224.

In an embodiment, the extraction unit 214 of the acoustic engine 108 is configured to extract or derive physiological condition signals from the input vibration signal.

In an embodiment, the amplification unit 216 of the acoustic engine 108 is configured to amplify the derived physiological condition signals to a desired frequency.

In an embodiment, the conversion unit 218 of the acoustic engine 108 is configured to determine and convert the physiological condition signals into physiological condition acoustic signals of a desired audio format.

In an embodiment, the processing unit 220 of the acoustic engine 108 may comprise one or more of microprocessors, circuits, and other hardware configured for processing. The processing unit 220 is configured to execute instructions stored in the memory unit 222 as well as communicate with at least one of: sensor devices 102, data capturing devices 104, data receiver modules 106, and user devices 110 through an input/output module via a communication module.

In an embodiment, the memory unit 222 of the acoustic engine 108 comprises one or more volatile and non-volatile memory components which are capable of storing data and instructions to be executed.

In an embodiment, the database 224 is configured to store one or more micro-voltage digital signals in a predetermined data storage format. The predetermined data storage format may comprise storing one or more datasets in a chronological order format, in the form of micro-voltage digital signal datasets.

In an embodiment of the present invention, the database 224 is configured to transmit the stored micro-voltage digital datasets corresponding to physiological parameters of the subject, to one or more other components of the system 100.

In an embodiment, the database 224 is also configured to save one or more stored physiological condition acoustic signals. These stored physiological condition acoustic signals may be further used for predicting future health issues of the user.

In an embodiment, the at least one user device 110 is configured to enable the at least one user to choose and hear their physiological condition acoustic signals. In an embodiment, the at least one user device 110 is configured to enable the at least one user to hear past physiological condition acoustic signals, as well as current and predicted physiological condition acoustic signals. The audible physiological conditions may comprise at least one of cardiac functions, heart beats and breathing of the at least one user.

In an embodiment, the system 100 may comprise as many user devices 110 as required by the users. The user devices 110 may comprise one or more wearable devices, mobile phones, tablets, PDA, smartphones, smart band, smart watch, laptop, computer, etc.

Further, the at least one user device 110 comprises a user application 226, a processing module 228, a memory module 230 and a communication module 232.

In an embodiment, the processing module 228 of the at least one user device 110 may comprise one or more of microprocessors, circuits, and other hardware configured for processing. The processing module 228 is configured to execute instructions stored in the memory module 230 as well as communicate with at least one of: sensor devices 102, data capturing devices 104, and data receiver modules 106, through an input/output module via the communication module 232.

In an embodiment, the memory module 230 of the at least one user device 110 comprises one or more volatile and non-volatile memory components which are capable of storing data and instructions to be executed.

In an embodiment, the user application 226 is configured to provide a user interface and dashboard for the at least one user to consume their physiological condition acoustic signals. Further, in case of any determined health issues related to the one or more physiological conditions of the at least one user, the user application 226 is configured to provide one or more alerts in the form of vibratory, audio, and visual alerts. Additionally, the user application 226 may be configured to alert one or more healthcare providers and designated contacts of the user.

In an embodiment, the functions of the acoustic engine 108 may be implemented in a local device such as the data receiver module 106, in order to provide a stand-alone system. In this case, the data receiver module 106 may be configured to generate and play the created acoustic signals by using on-hardware processing (through chips) and edge-computing.

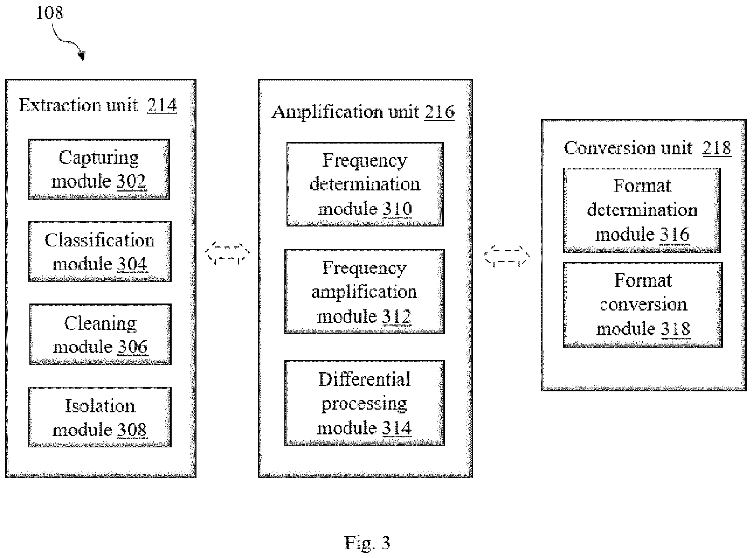

Fig. 3 depicts/illustrates a detailed block diagram of components of the acoustic engine 108, in accordance with an embodiment.

In an embodiment, the extraction unit 214 comprises a capturing module 302, a classification module 304, a cleaning module 306 and an isolation module 308.

In an embodiment, the capturing module 302 comprised within the extraction unit 214 is configured to select data windows from the vibration signal to extract data clusters which are representations of specific physiological conditions. The specific physiological conditions may comprise one or more of heartbeat, and breath, among others.

In an embodiment, the capturing module 302 utilizes a moving data window to capture characteristics of individual specific physiological conditions such as heart beats, from the vibration signal. Further, a data window of 4-49ms may be used for the same.

In an embodiment, the capturing module 302 is configured to extract data windows and reject any data windows which contain disturbances such as body motions of the user. Further, the capturing module 302 is configured to generate templates and group the templates into clusters to recognize clusters comprising the specific physiological condition, such as heartbeats.

In an embodiment, the classification module 304 comprised within the extraction unit 214 is configured to classify one or more portions of the vibration signal into at least one of clean instance, movement instance and artifacts.

In an embodiment, the cleaning module 306 comprised within the extraction unit 214 is configured to remove or clean at least one of the movement instance 404 and artifact 406, by using a clustering algorithm.

In an embodiment, the clustering algorithm determines whether a 1-second instance of the vibration signal 402 comprises a movement instance 404. Further, the cleaning module 306 is configured to remove the movement instances 404 and artifacts 406.

In an embodiment, the isolation module 308 comprised within the extraction unit 214 is configured to isolate one or more physiological condition signals from the cleaned vibration signal 402.

In an embodiment, the amplification unit 216 comprises a frequency determination module 310, a frequency amplification module 312, and a differential processing module 314.

In an embodiment, the frequency determination module 310 comprised within the amplification unit 216 is configured to determine at least one desired frequency of the output physiological condition acoustic signals. The at least one desired frequency may be calculated automatically, based on the frequency of the isolated physiological condition signals. Alternatively, the desired frequency may be determined based on one or more instructions stored in the acoustic engine 108 or received as a user input from the at least one user device 110.

In an embodiment, advantageously, the frequency determination module 310 is configured to eliminate any human speech and ambient sound, to enables a non-intrusive, contact-less system that works in a user's personal spaces without raising any privacy concerns.

In an embodiment, advantageously, the frequency determination module 310 is configured to implement hardware components such as at least one of piezoelectric elements under a dampening material like a mattress, sampling at lower rates, and software filters, to eliminate any high frequency sounds of human voices and other ambient noises.

Advantageously, the implementation of piezoelectric elements, sampling at lower rates, and software filters prevents any speech data or personal data from being recorded by the system 100, thus enabling the non-intrusive function of the system 100.

In an embodiment, a large section of data can be represented in a short acoustic signal or audio file. As an example, a larger period of time such as a day, week or month of the user's physiological conditions, may be represented in a created acoustic signal.

In an embodiment, the frequency amplification module 312 comprised within the amplification unit 216 is configured to amplify the physiological condition signals to a desired frequency. Further, one or more available libraries may be used to save the signals with desired higher frequency values.

In an embodiment, the frequency amplification module 312 may calculate and execute the amplification automatically by resampling and amplifying the physiological condition signal based on a desired frequency range.

In an embodiment, the differential processing module 314 is configured to generate one or more created acoustic signals by combining, merging or layering at least one of multiple signals, layers and digital markers that depict clinical issues related to the one or more physiological conditions of the user's body.

Further, the created acoustic signals may capture the one or more physiological conditions such as low ejection fraction, high ejection fraction, heart murmurs, lung crackles, coughs, wheezing, and fluid in any organs, among others.

In an embodiment, the conversion unit 218 comprises a format determination module 316, and a format conversion module 318.

In an embodiment, the format determination module 316 comprised within the conversion unit 218 is configured to determine at least one desired audio file format for the output physiological condition acoustic signals. The at least one desired audio file format may be chosen automatically, may be determined based on one or more instructions stored in the acoustic engine 108, or received as a user input from the at least one user device 110.

In an embodiment, the format conversion module 318 comprised within the conversion unit 218 is configured to convert the physiological condition signals into physiological condition acoustic signals of the desired audio file format. The desired audio format may comprise at least one of waveforms, array formats, mixed formats, .aif, .snd, Audio for Unix formats, .ir, .dxr, .mid, .mod, and MPEG audio formats, among others.

Hence, advantageously, the disclosed system provides a reliable way to measure create and monitor acoustic signals to monitor the one or more physiological conditions of a single user over a long duration, and hence provides immense benefits for personalized non-intrusive healthcare, which does not require any human intervention.

In an embodiment, the alert may be communicated to one or more users, user devices 110, designated contacts of a user, healthcare professionals, doctors, nurses, and clinicians, among others.

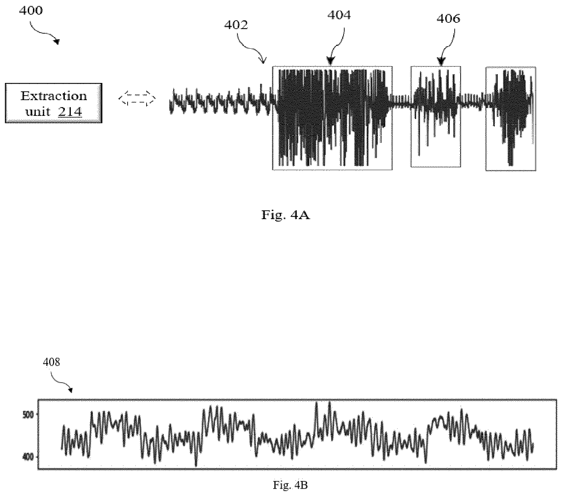

Fig. 4A depicts/illustrates an exemplary vibration signal 402 being cleaned by an extraction unit 214, in accordance with an embodiment.

In an embodiment, the classification module 304 comprised within the extraction unit 214 is configured to determine and classify one or more portions or seconds of the vibration signal 402 into at least one of a clean instance, movement instances 404 and artifacts 406 present in the vibration signal 402. The determined movement instances 404 and artifacts 406 are subsequently cleaned or removed from the BCG signal 402 by the cleaning module 306.

Fig. 4B depicts/illustrates a cleaned raw signal 408, in accordance with an embodiment.

In an embodiment, after the cleaning module 306 has removed the movement instances 404 and artefacts 406 from the vibration signal 402, the isolation module 308 is further used to derive at least one physiological condition signal from the raw input vibration signal 408.

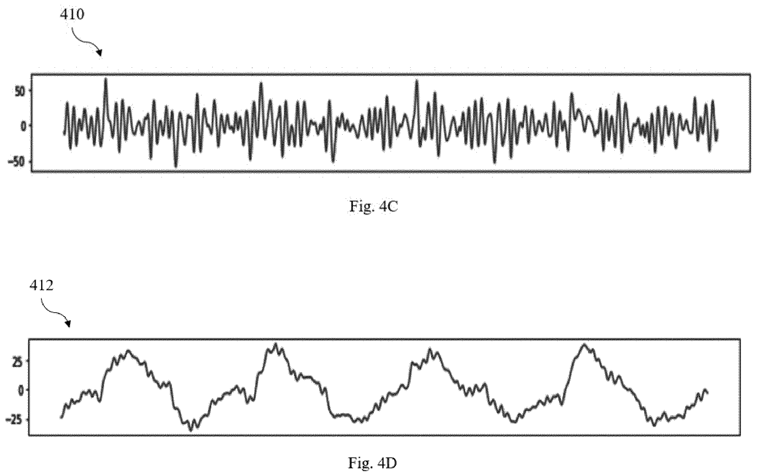

Fig. 4C depicts/illustrates an isolated heart signal 410 of a user, in accordance with an embodiment.

Fig. 4D depicts/illustrates an isolated breathing signal 412 of a user, in accordance with an embodiment.

In an embodiment, the heart signal 410 and the breathing signal 412 have been derived from the raw signal 408 by the isolation module 308. The isolation module 308 may use one or more filters to derive the heart signal 410 and the breathing signal 412.

In an embodiment, the isolation module 308 uses at least one of a Butterworth bandpass filter, Chebyshev and Bessel filters, to isolate at least one physiological condition signal from the raw signal 408.

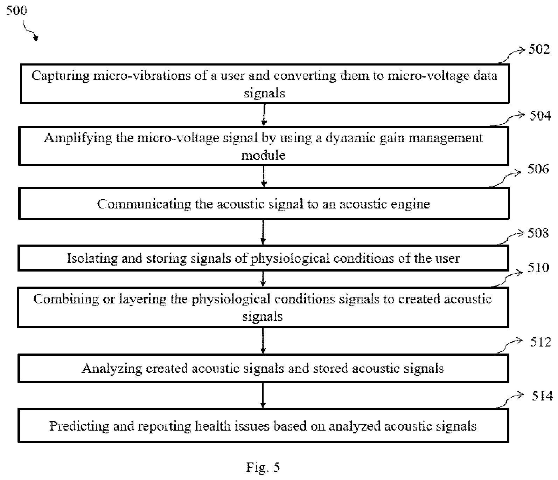

Fig. 5 illustrates a method for non-intrusive monitoring of the one or more physiological conditions through acoustic signals, in accordance with an embodiment.

The method begins with capturing micro-vibrations of a user and converting them to micro-voltage data signals, as depicted at step 502. Subsequently, the method 500 discloses amplifying the micro-voltage signal by using a dynamic gain management module, as depicted at step 504.

Thereafter, the method 500 discloses communicating the signal to an acoustic engine, as depicted at step 506. Further, the method 500 discloses isolating and storing signals of one or more physiological conditions of the patient, as depicted at step 508. The method 500 then discloses combining or layering physiological condition signals to created acoustic signals, as depicted at step 510.

Further, the method 500 discloses analyzing created acoustic signals and stored acoustic signals, as depicted at step 512. Subsequently, the method 500 discloses analyzing, predicting and reporting health issues based on analyzed acoustic signals, as depicted at step 514.

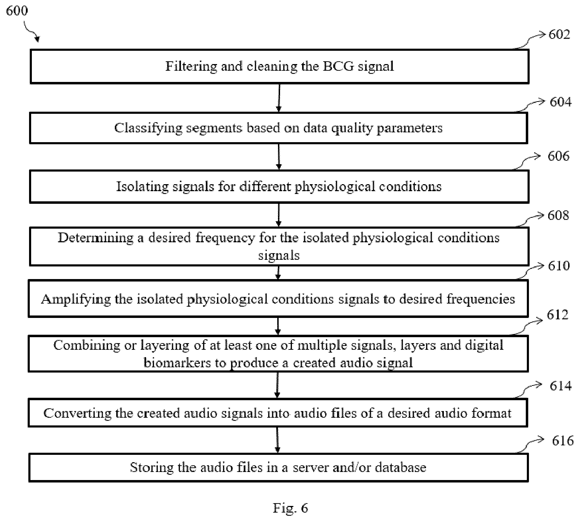

Fig. 6 illustrates a method 600 for extraction of acoustic signals and generation of created audio files from a vibration signal, in accordance with an embodiment.

The method begins with filtering and cleaning the vibration signal, as depicted at step 602. Subsequently, the method 600 discloses classifying segments based on data quality parameters, as depicted at step 606. Thereafter, the method 600 discloses isolating signals for different physiological conditions, as depicted at step 606.

Further, the method 600 discloses determining a desired frequency for the isolated physiological condition signals, as depicted at step 608. The method 600 then discloses amplifying the isolated physiological condition signals to desired frequencies, as depicted at step 610.

Further, the method 600 discloses combining or layering of at least one of multiple signals, layers and digital biomarkers to produce a created acoustic signal, as depicted at step 612. The method 600 then discloses converting the amplified signals into audio files of the desired audio format, as depicted at step 614. Additionally, the method 600 discloses storing the audio files in a server and/or database, as depicted at step 616.

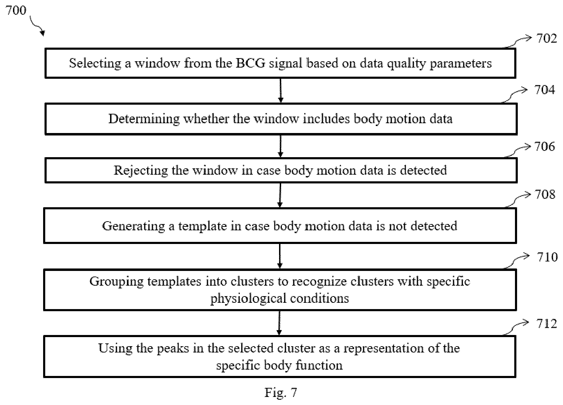

Fig. 7 illustrates a method 700 for capturing data windows in a vibration signal, in accordance with an embodiment.

The method begins with selecting a window from the vibration signal based on data quality parameters, as depicted at step 702. Further, the method 700 discloses determining whether the window includes body motion data, as depicted at step 704. Thereafter, the method 700 discloses rejecting the window in case body motion data is detected, as depicted at step 706.

Further, the method 700 discloses generating a template in case body motion data is not detected, as depicted at step 708. The method 700 discloses grouping templates into clusters to recognize clusters with specific physiological conditions, as depicted at step 710.

Further, the method 700 discloses using the peaks in the selected cluster as a representation of the specific physiological condition, as depicted at step 712.

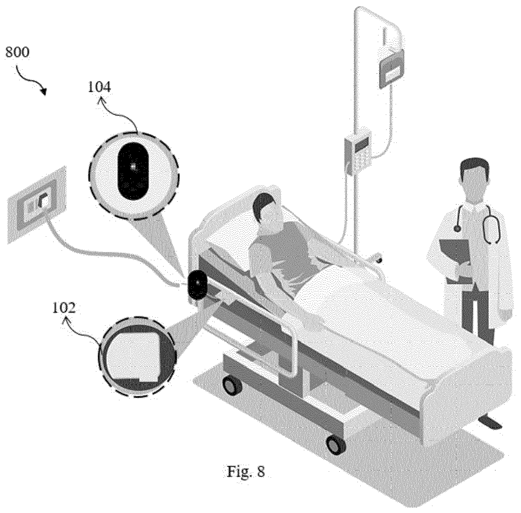

Fig. 8 depicts/illustrates an exemplary placement 800 of the at least one sensor device 102 and the at least one data capturing device 104, in accordance with an embodiment. In this example, the at least one sensor device 102 is used in a hospital/ clinic/ medical care environment, where the at least one sensor device 102 may be placed under the mattress or bed of the user, who may be a patient suffering from one or more health issues. The at least one sensor device 102 can be used to monitor the physiological condition acoustic signals of the patient in a non-intrusive, real-time continuous manner, without requiring any human intervention from nurses, doctors or healthcare professionals.

In an embodiment, the at least one data capturing device 104 may be positioned in the vicinity of the patient, as depicted in the figure. In an embodiment, the connection between the at least one sensor device 102 and the at least one data capturing device 104 may be a wired or a wireless connection, depending on the environment and user requirement. Further, the at least one data capturing device 104 may use an in-built battery source, or a wired battery source, for its functioning.

In an embodiment, the implementation of the disclosed system 100 and methods 500, 600, 700 as a computer program may comprise an intangible form by using wireless communication.

In an embodiment, the disclosed system 100 and methods 500, 600 may be suitably implemented as a computer program to be implemented by a computing device. The method described herein may be typically implemented as a set of instructions to be executed by a computer, laptop, server, cloud server, or other similar computing devices.

In an embodiment, the set of program instructions may comprise a series of computer readable codes saved in a tangible storage comprising at least one of a computer readable storage medium such as a ROM, EPROM, flash drive, or hard disk; or may be transmitted to the computing device through wired or wireless means.

The advantages of the current invention include providing a simple, autonomous and cost-effective system and method for non-intrusive monitoring of the one or more physiological conditions through acoustic signals.

An additional advantage is that the technology reduces the skill level required to monitor physiological conditions, as it does not require trained medical practitioners to analyze the vibration signal of the at least one user to determine one or more physiological conditions of the at least one user.

Another additional advantage is that the disclosed system is less or non-intrusive and very comfortable when implemented in the vicinity of the at least one user or patient. Additionally, the system can also be used in a daily or continuous manner in order to autonomously detect rapid changes in physiological conditions, without human intervention.

Applications of the current invention include remote patient monitoring and prediction of physiological conditions. The described system can be used in hospitals, clinics, camp set-ups, homes, humanitarian shelters, etc.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described here.

## Claims

1. A system (100) for non-intrusive monitoring of physiological conditions through created acoustic signals, comprising:
at least one sensor device (102) configured to capture one or more analog micro-vibrations from at least one user and convert the micro-vibrations into at least one digital data signal;
at least one data capturing device (104) configured to receive and process the at least one digital data signal from the at least one sensor device (102); and
an acoustic engine (108) configured to convert the at least one processed digital data signal from the at least one data capturing device (104) into one or more frequency-based physiological acoustic signals, wherein the created frequency-based physiological acoustic signals are processed to determine one or more physiological conditions of the at least one user.

2. The system (100) as claimed in claim 1, wherein the at least one sensor device (102) comprises at least one sensor array, wherein the sensor array comprises one or more of: piezoelectric sensors, and vibroacoustic sensors.

3. The system (100) as claimed in claim 1, wherein the at least one sensor device (102) is configured to comprise at least one of:
a strengthened outer cover for protecting and covering inner components of the at least one sensor device (102), wherein the strengthened outer cover comprises a durable material; and
an electromagnetic shielding fabric that encases the sensor array, wherein the electromagnetic shielding fabric increases the signal to noise ratio of the detected signal and prevents any interference or digital noise, from any erroneous noise from an environment of the at least one sensor device (102).

4. The system (100) as claimed in claim 1, wherein vibrations captured by the at least one sensor device (102) comprises at least one of mechanical, and force-based signals comprising at least one of vibrations, microvibrations, macrovibrations, ballistocardiograph (BCG vibration), seismo-cardiographs, and impedance signals associated with physiological parameters of the user's body.

5. The system (100) as claimed in claim 1, wherein the at least one data capturing device (104) comprises a data capturing engine (202) configured to receive the at least one digital data signal from the at least one sensor device (102) and process the at least one digital data signal in order to derive amplified data signals.

6. The system (100) as claimed in claim 5, wherein the data capturing engine (202) comprises:
a data acquisition unit (204) configured to record or store the received micro-voltage digital signal in a predetermined data format, wherein the data acquisition unit (204) that is communicatively coupled to the at least one sensor device (102) is configured to:
store the at least one received digital data signals based on at least one of user accounts, database segments for different users, different geographical applications, and different applications of the at least one sensor device (102);
communicate the at least one stored digital data signal to the conditioning unit (206); and
utilize one or more encryption techniques to ensure the communication of secure, encrypted, ordered data to a cloud-based storage or processing system;
a conditioning unit (206) configured to amplify the received micro-voltage digital signals in order to maximize resolution of the digital signals to obtain a desired amplified signal, wherein the conditioning unit (206) is configured to:
determine the amplified signal for efficient detection of the user's physiological parameters;
maximize the resolution of the digital signals by ensuring prevention of any data loss which occur due to loss of the signal;
amplify the at least one digital data signal based on strength of the at least one received digital data signal;
automatically calibrate and select the amplification option based on a sensed proximity of the at least one sensor device (102) to the user; and
communicate the optimized amplified signal to the transmission unit (208); and a transmission unit (208) configured to transmit the amplified digital data signal to one or more of the acoustic engines (108), a database (118), and a data receiver module (106).

7. The system (100) as claimed in claim 1, wherein the acoustic engine (108) comprises:
an extraction unit (214), configured to extract or derive physiological condition signals from the one or more analog micro-vibrations;
an amplification unit (216) configured to amplify the derived physiological condition signals to a desired frequency by using a dynamic gain management module; and
a conversion unit (218) configured to determine desired audio file formats and convert the amplified physiological condition signals into audio files.

8. The system (100) as claimed in claim 7, wherein the extraction unit (214) comprises:
a capturing module (302) select one or more data windows from a vibration signal (402) to extract data clusters which are representations of specific physiological conditions, wherein the capturing module (302) is configured to:
utilize a moving data window to capture characteristics of individual specific physiological conditions;
extract data windows and reject any data windows which contain disturbances such as body motions of the user; and
generate templates and group the templates into clusters to recognize clusters comprising the specific physiological condition, such as heartbeats;
a classification module (304) configured to classify one or more portions of the vibration signal into at least one of clean instance, movement instance and artifacts;
a cleaning module (306) configured to remove or clean at least one of the movement instance (404) and artifact (406) by using a clustering algorithm to determine whether a 1-second instance of the vibration signal (402) comprises the movement instance (404); and
an isolation module (308) configured to isolate one or more physiological condition signals from the cleaned vibration signal (402).

9. The system (100) as claimed in claim 7, wherein the amplification unit (216) comprises:
a frequency determination module (310) configured to determine at least one desired frequency of the output physiological condition acoustic signals;
a frequency amplification module (312) configured to calculate and execute an amplification automatically by resampling and amplifying the physiological condition signal based on a desired frequency range; and
a differential processing module (314) configured to generate one or more created acoustic signals by combining, merging or layering at least one of multiple signals, layers and digital markers that depict clinical issues related to physiological conditions of the user's body.

10. The system (100) as claimed in claim 9, wherein the frequency determination module (310) is configured to:
determine the desired frequency based on at least one or more instructions stored in the acoustic engine (108), or the instructions received as a user input from the at least one user device (110); and
eliminate any high frequency sounds of human speech and ambient sound, using at least one of piezoelectric elements under a dampening material, sampling at lower rates, and software filters, to eliminate any human voices and other ambient noises.

11. The system (100) as claimed in claim 7, wherein the conversion unit (218) comprises:
a format determination module (316) configured to determine at least one desired audio file format for the output physiological condition acoustic signals; and
a format conversion module (318) configured to convert the physiological condition signals into physiological condition acoustic signals of the desired audio file format.

12. The system (100) as claimed in claim 1, wherein the acoustic engine (108) is configured to analyze and compare the created frequency-based physiological acoustic signals with stored physiological condition acoustic signals, in order to predict health issues of the at least one user and to generate one or more alerts based on the health issues, and wherein the one or more alerts are received by using at least one user device (110) that comprises a user application (226).

13. The system (100) as claimed in claim 1, wherein the created frequency-based physiological condition acoustic signals comprise signals related to at least one of physiological conditions and biomarkers of the user's body, and wherein the physiological conditions comprise at least one of: low ejection fraction, high ejection fraction, heart murmurs, lung crackles, coughs, wheezing, and fluid in any organs.

14. The system (100) as claimed in claim 12, wherein the stored physiological condition acoustic signals comprise previously gathered data of physiological conditions of previous users.

15. A method (500) for non-intrusive monitoring of physiological conditions through acoustic signals, comprising:
capturing one or more analog micro-vibrations of at least one user and converting them to at least one digital data signal by using at least one sensor device (102);
receiving and processing the at least one digital data signal from the at least one sensor device (102) by using at least one data capturing device (104);
amplifying the at least one digital data signal by using a dynamic gain management module;
communicating the at least one digital data signal to an acoustic engine (108) by using a communication network (112);
isolating and storing signals of one or more physiological conditions of the patient by using an isolation module (308) in extraction unit (214) of the acoustic engine (108);
combining or layering the one or more physiological condition signals to created acoustic signals by using a differential processing module (314) in amplification unit (216) of the acoustic engine (108);
analyzing created acoustic signals and stored acoustic signals by using the acoustic engine (108); and
predicting and reporting health issues based on analyzed acoustic signals by using the acoustic engine (108).

16. The method (500) as claimed in claim 15, comprising configuring the data capturing engine (202) for:
recording or storing the received micro-voltage digital signal in a predetermined data format, by using a data acquisition unit (204);
amplifying the received micro-voltage digital signals in order to maximize resolution of the digital signals to obtain a desired amplified signal, by using a conditioning unit (206); and
transmitting the amplified digital data signal to one or more of the acoustic engine (108), a database (118), and a data receiver module (106), by using a transmission unit (208).

17. The method (500) as claimed in claim 16, comprising configuring the data acquisition unit (204) that is communicatively coupled to the at least one sensor device (102) for:
storing the at least one received digital data signal based on at least one of user accounts, database segments for different users, different geographical applications, and different applications of the at least one sensor device (102);
communicating the at least one stored digital data signal to the conditioning unit (206); and
utilizing one or more encryption techniques to ensure the communication of secure, encrypted, ordered data to a cloud-based storage or processing system.

18. The method (500) as claimed in claim 16, comprising configuring the conditioning unit (206) for:
determining the amplified signal for efficient detection of the user's physiological parameters;
maximizing the resolution of the digital signals by ensuring prevention of any data loss which may occur due to loss of the signal;
amplifying the at least one digital data signal based on strength of the at least one received digital data signal;
calibrating and selecting the amplification option based on a sensed proximity of the at least one sensor device (102) to the at least one user automatically; and
communicating the optimized amplified signal to the transmission unit (208).

19. The method (500) as claimed in claim 15, comprising configuring the acoustic engine (108) for extraction of acoustic signals and generation of created audio files from a vibration signal, wherein the method comprises:
filtering and cleaning the vibration signal (402) by using an isolation module (308) and a cleaning module (306) in an extraction unit (214) of an acoustic engine (108);
classifying segments based on data quality parameters by using classification module (304) in an extraction unit (214) of an acoustic engine (108);
isolating signals for different physiological conditions by using an isolation module (308) in an extraction unit (214) of the acoustic engine (108);
determining a desired frequency for the isolated physiological condition signals by using frequency determination module (310) in an amplification unit (216) of the acoustic engine (108);
amplifying the isolated physiological condition signals to desired frequencies by using frequency amplification module (312) in an amplification unit (216) of the acoustic engine (108);
combining or layering of at least one of multiple signals, layers and digital biomarkers to produce a created acoustic signal by using a differential processing module (314) in amplification unit (216) of the acoustic engine (108);
converting the amplified signals into audio files of the desired audio format by using a conversion unit (218); and
storing the audio files in a server and/or database.

20. The method (500) as claimed in claim 15, comprising configuring the acoustic engine for capturing data windows in a vibration signal, wherein the method comprises:
selecting one or more windows from a vibration signal (402) based on data quality parameters by using a capturing module (302) in an extraction unit (214) of an acoustic engine (108);
determining whether the windows comprises body motion data by using the capturing module (302) in the extraction unit (214) of the acoustic engine (108);
rejecting the window in case body motion data is detected by using the capturing module (302) in the extraction unit (214) of the acoustic engine (108);
generating a template in case body motion data is not detected by using the capturing module (302) in the extraction unit (214) of the acoustic engine (108);
grouping templates into clusters to recognize clusters with specific physiological conditions by using the capturing module (302) in the extraction unit (214) of the acoustic engine (108); and
using the peaks in the selected cluster as a representation of the specific physiological condition by using the capturing module (302) in the extraction unit (214) of the acoustic engine (108).
